Europäisches Patentamt

(19) European Patent Office  (11) Publication number: **0 116 726**

Office européen des brevets  **A1**

(12) **EUROPEAN PATENT APPLICATION**

(21) Application number: **83201669.5**  (51) Int. Cl.³: **A 61 M 5/16**

(22) Date of filing: **24.11.83**

(30) Priority: **25.11.82 NL 8204598**

(43) Date of publication of application:
**29.08.84 Bulletin 84/35**

(84) Designated Contracting States:
**AT BE CH DE FR GB IT LI LU NL SE**

(71) Applicant: **MEDICA B.V.**
**Lederstraat 1**
**NL-5223 AW Den Bosch(NL)**

(72) Inventor: **Van Veen, Jan**
**21 Prof. Rutgerstraat**
**NL-5224 JN Den Bosch(NL)**

(74) Representative: **Hoorweg, Petrus Nicolaas et al,**
**OCTROOIBUREAU ARNOLD & SIEDSMA Sweelinckplein**
**1**
**NL-2517 GK The Hague(NL)**

(54) Aerating device for a fluid container for medical purposes.

(57) A device for aerating a fluid container (1) for medical purposes, wherein a housing (7) has a passage, one end of which adjoins a tube (5) connected with the flask (1), whereas the other end opens out in an air chamber (8) formed by a square prism, one wall of which being pervious to air in order to maintain a constant pressure in said flask (1) thus being able to vary the closing of said fluid very precisely.

EP 0 116 726 A1

./...

FIG.1

Aerating device

-------------------------------------------------------

The invention relates to a device for aerating a fluid container for medical purposes.

It is common practice to suspend a fluid container for an intravenous fluid with the outlet port downwards, whilst a tube for the evacuation of the fluid hangs down. In addition it is common practice to use a second, flexible tube bent upwards from said lower port to form an air inlet. The tube is closed by an air-pervious plug. The drawback of such an arrangement is that relatively much fluid is present in the aerating tube so that first subatmospheric pressure has to be created in the flask to admit air. Particularly in the case of very precise dosing this subatmospheric pressure may have a highly adverse effect due to the oscillating movement of the fluid repeatedly flowing back in the aerating tube because the subatmospheric pressure varies with the height of the column in the aerating tube.

The invention has for its object to avoid such a drawback and to provide for this purpose a device of the kind set forth in the preamble which is distinguished by a housing having a passage, one end of which adjoins a tube connected with the flask, whereas the other end opens out in an air chamber, one wall of which is pervious to air.

Owing to the use of an air chamber the amount of fluid flowing into the device is only small at the beginning of the take-up so that the variation in subatmospheric pressure is limited.

In order to fully evacuate fluid from the chamber to allow undisturbed air supply the passage opens out in the lower part of the air chamber in accordance with the invention. Thus the oscillating movement of the fluid is further limited and hence also the variation in subatmospheric pressure in the fluid container. In a preferred embodiment the air chamber is mainly formed by a square prism, the passage extending diagonally along the same. In this advantageous form the fluid will accumulate in the lower tip of the air chamber so that the air chamber is exhausted substantially completely.

Preferably the square boundary wall of the air chamber is pervious to air so that an optimum supply of air to the air chamber is ensured.

The wall is preferably formed by a filter of a hydrophobic material by which contamination of the fluid or the air above the fluid is avoided.

The invention will be described more fully with reference to two embodiments.

The drawing shows in:

Fig. 1 a perspective view of a schematic arrangement of a fluid container with an aerating device embodying the invention,

Fig. 2 a perspective view of the aerating device used in fig. 1, part of the housing being broken away,

Fig. 3 a vertical sectional view of an aerating device in a second embodiment,

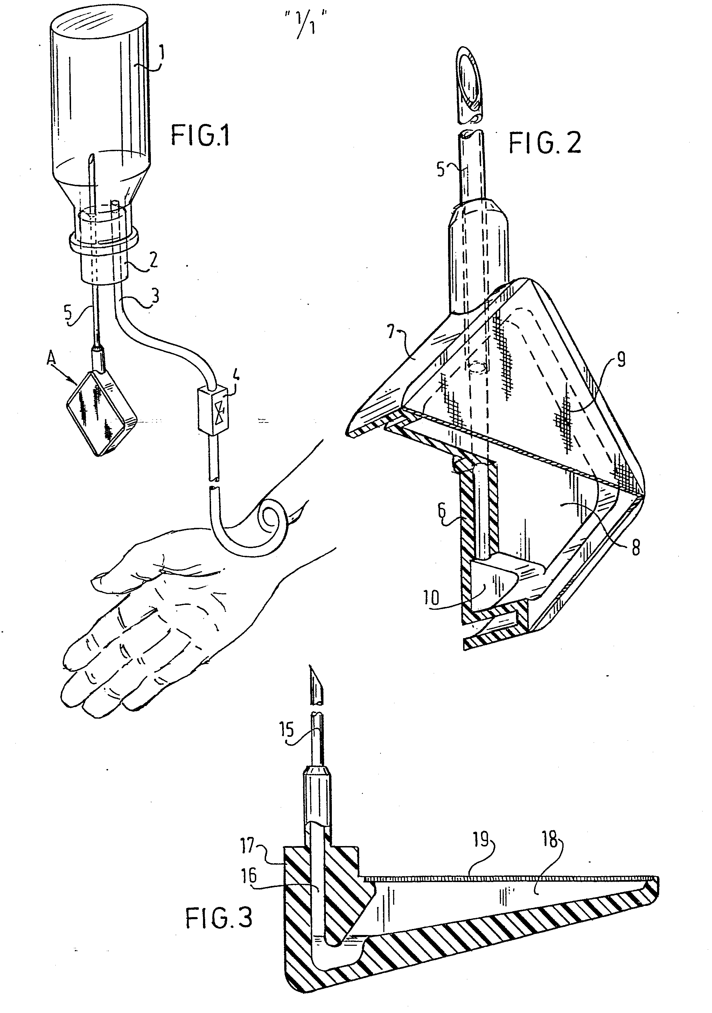

Reference numeral 1 in fig. 1 designates the flask containing a fluid for medical purposes, for example, an infusion to be administered intravenously.

The neck of the flask is closed by a plug 2 through which is passed a tube 3 leading through a dripping set 4 towards the patient, whose lower arm is shown. Through the plug 2 is furthermore passed a tube 5 leading to an aerating device A.

The aerating device A embodying the invention is shown in detail in fig. 2, in which the tube 5 opens out in a passage 6 in a housing 7. The housing has the shape of a rhombic prism, the diagonal of which is vertical. In the housing is recessed an air chamber 8 which is covered at the front by a hydrophobic filter 9 forming the wall of the air chamber 8. On the rear side, that is to say the side of the air chamber 8 remote from the hydrophobic filter 9 the passage 6 diagonally extends to the lower part 10 of the air chamber, which lower part is furthermore formed by the lateral boundary walls of the air chamber extending towards one another. The lower part 10, therefore, has a small fluid capacity as compared with the air chamber 8.

The device described above operates as follows: After mounting the tube 5, which has a bevelled cut edge in known manner, the fluid will flow from the flask 1 through the tube 5 and the passage 6 into the air chamber 8, which can take place without hindrance because the air contained in the air chamber 8 is expelled through the filter 9.

As soon as a fluid is demanded through the tube 3 subatmospheric pressure will form in the flask 1 so that fluid is sucked from the air chamber 8 through the passage 6 and the tube 5. As soon as the air chamber 8 is completely evacuated, air can penetrate into the passage 6 and the tube 5, which prevents further formation of subatmospheric pressure in the flask 1.

In view of the relatively small capacity of the air chamber or the lower part 10 thereof, substantially all fluid will be sucked out of the air chamber so that practically the whole filter is free of residual fluid. The relatively large filter surface 9 ensures undisturbed admission of air so that excessive subatmospheric pressure in the flask 1 is avoided. The subatmospheric pressure in the flask 1 required to start the supply of air is so small that the oscillating effect of the existing aerating systems is avoided.

Fig. 3 shows am embodiment in which the housing 17 has a horizontal air chamber 18 provided with a horizontally extending filter 19. The passage 16 from the tube 15 extends initially in vertical direction in the housing 17 and bends on the underside into the lowermost part of the air chamber 18.

This embodiment operates in the same manner as that of fig. 2, since also in this case at the demand or air the air chamber is first fully evacuated so that no residual fluid will remain in the device.

The invention is not limited to the embodiments described above. It is noted that the housing 7 may form part of a plug 2 or a tube 3 of a fluid flask. In this way an integrated tapping unit can be formed for a flask.

-----

WHAT IS CLAIMED IS:

1. A device for aerating a fluid container for medical purposes characterized by a housing with a passage, one end of which adjoins a tube connected with a flask, whereas the other end opens out in an air chamber, one wall of which is pervious to air.

2. A device as claimed in claim 1, characterized in that the passage opens out in the lowermost part of the air chamber.

3. A device as claimed in claims 1 and 2, characterized in that the air chamber is mainly formed in the shape of a square prism, the passage extending diagonally along the same.

4. A device as claimed in claims 1 to 3, characterized in that the air-pervious wall is in a vertical position.

5. A device as claimed in claims 1 to 4, characterized in that the filter consists of a hydrophobic material.

---

0116726

"1/1"

FIG.1

FIG.2

FIG.3

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| X | US-A-3 157 481 (A. BUJAN) <br> * Figures; column 1, lines 14-22; column 2, lines 8-54 * | 1,2,5 | A 61 M 5/16 |
| X | FR-A-1 279 076 (J. PFIRMMER & CO.) <br> * Figure; page 2, right-hand column, paragraph 5 - page 3, left-hand column, last paragraph * | 1,2 | |
| X | FR-A-2 246 281 (L. MAYETHEL) <br> * Figures; page 2, line 38 - page 3, line 18 * | 1,2 | |
| A | US-A-4 009 714 (K. HAMMER) <br> * Figure 6; column 5, lines 13-21 * | 1-5 | TECHNICAL FIELDS SEARCHED (Int. Cl. 3) |
| A | US-A-4 177 149 (D. ROSENBERG) | 1,3,5 | A 61 M |

The present search report has been drawn up for all claims

| Place of search | Date of completion of the search | Examiner |
|---|---|---|
| THE HAGUE | 07-03-1984 | VEREECKE A. |

EPO Form 1503. 03.82